(19) Europäisches Patentamt
European Patent Office
Office européen des brevets

(11) Veröffentlichungsnummer: **0 056 956**
**B1**

# (12) EUROPÄISCHE PATENTSCHRIFT

(45) Veröffentlichungstag der Patentschrift: **27.11.85**

(51) Int. Cl.⁴: **C 07 C 143/822,** A 61 K 31/18

(21) Anmeldenummer: **82100238.3**

(22) Anmeldetag: **14.01.82**

(54) **Neue Indanyl-Derivate, ihre Herstellung und Verwendung.**

(30) Priorität: **27.01.81 DE 3103372**

(43) Veröffentlichungstag der Anmeldung: **04.08.82 Patentblatt 82/31**

(45) Bekanntmachung des Hinweises auf die Patenterteilung: **27.11.85 Patentblatt 85/48**

(84) Benannte Vertragsstaaten: **AT BE CH DE FR GB IT LI LU NL SE**

(56) Entgegenhaltungen: **EP-A-0 009 554**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Schröder, Eberhard, Dr., Verstorben (DE)**
Erfinder: **Rufer, Clemens, Dr., Westhofener Weg 46, D-1000 Berlin 38 (DE)**
Erfinder: **Böttcher, Irmgard, Dr., Frobenstrasse 46, CH-4000 Basel (CH)**
Erfinder: **Kapp, Joachim-Friedrich, Dr., 40 Dogwood Lane, Princeton, NJ 08546 (US)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Indanyl-Derivate gemäss Patentanspruch 1, ein Verfahren zu ihrer Herstellung gemäss Patentanspruch 9 und pharmazeutische Präparate gemäss Patentanspruch 8, die diese Indanyl-Derivate als Wirkstoff enthalten.

Das erfindungsgemässe Verfahren zur Herstellung der neuen Indanyl-Derivate kann unter den Bedingungen durchgeführt werden, die in der Europäischen Patentanmeldung 0 009 554 beschrieben sind. Gegenüber den in dieser Anmeldung beschriebenen Indanyl-Derivaten (mit Ausnahme von Verbindungen der allgemeinen Formel I mit $R_2$ und $R_3$ in der Bedeutung einer Oximinogruppe, die vorzugsweise als Zwischenprodukte verwendet werden) zeichnen sich die erfindungsgemässen Verbindungen durch eine überlegene Wirksamkeit aus, wie die Ergebnisse des nachfolgend beschriebenen Adjuvans Arthritis-Tests zeigen:

Es werden weibliche und männliche Ratten des Stammes Lewis (LEW) in der Gewichtsspanne zwischen 110–190 g verwendet. Die Tiere erhalten Trinkwasser und Altromin-Pressfutter ad libitum.

Für jede Dosisgruppe werden 10 Ratten eingesetzt.

Mycobacterium butyricum der Firma Difko, Detroit, wird als Reizmittel verwandt. Eine Suspension von 0,5 mg Mycobacterium butyricum in 0,1 ml dünnflüssigem Paraffin (DAB 7) wird in die rechte Hinterpfote subplantar injiziert.

Die Testsubstanzen werden vom 11. Versuchstag an täglich über 4 Tage oral gegeben. Die Substanzen werden als klare wässrige Lösung oder als Kristallsuspension unter Zusatz von Myrj 53 (85 mg %) in isotonischer Natriumchlorid-Lösung verabreicht.

Die Ratten werden in bezug auf ihr Körpergewicht möglichst gleichmässig in verschiedene Gruppen eingeteilt. Nach plethysmographischer Volumenmessung der rechten Hinterpfote wird in diese subplantar 0,1 ml Adjuvans injiziert.

Die rechten Hinterpfoten werden vom 14. Versuchstag bis zu Versuchsende gemessen. Die Versuchsdauer beträgt drei Wochen.

Bestimmt wird die Abheilung der rechten Pfote des Tieres in Abhängigkeit von der applizierten Dosis an Testsubstanz.

Die nachfolgende Tabelle zeigt die in diesem Test erhaltenen Ergebnisse der erfindungsgemässen Verbindungen 3 bis 5 im Vergleich zu den aus der Europäischen Patentanmeldung 0 009 554 vorbekannten strukturanalogen Indanyl-Derivaten 1 und 2. Die Ergebnisse zeigen, dass die erfindungsgemässen Verbindungen bei so niedriger Dosierung gut wirksam sind, bei der die Vergleichssubstanzen praktisch keine Wirksamkeit zeigen.

| Nr. | Substanz | Substanz mg/kg Tier | % Abheilung der rechten Pfote |
|---|---|---|---|
| 1 | N-[6-(4-Fluorphenoxy)-5-indanyl]-methansulfonamid | 4 × 0,1 | 0 |
| | | 4 × 0,3 | 0 |
| 2 | N-[6-(2,4-Dichlorphenoxy)-5-indanyl]-methansulfonamid | 4 × 0,1 | 0 |
| | | 4 × 0,3 | 3 |
| 3 | N-[6-(2,4-Difluorphenoxy)-5-indanyl]-methansulfonamid | 4 × 0,1 | 33 |
| | | 4 × 0,3 | 40 |
| 4 | N-Acetyl-N-[6-(2,4-difluorphenoxy)-5-indanyl]-methansulfonamid | 4 × 0,1 | 28 |
| | | 4 × 0,3 | 38 |
| 5 | 6-(2,4-Difluorphenoxy)-5-methylsulfonyl-1-indanon | 4 × 0,1 | 36 |
| | | 4 × 0,3 | 42 |

Somit eignen sich die neuen Verbindungen in Kombination mit den in der galenischen Pharmazie üblichen Trägermitteln zur Behandlung von Erkrankungen des rheumatischen Formenkreises (wie die Ostearthritis oder ankylosierenden Spondalitis) Asthma bronchiale, Heufieber u.a..

Bemerkenswert ist ferner, dass sich die Imidazol-Derivate der allgemeinen Formel I gemäss Anspruch 1 darüberhinaus auch zur Behandlung von Migräne und von Dysmenorrhö eignen und das Thromboserisiko mindern.

Überraschenderweise gibt es unter den erfindungsgemässen Indanyl-Derivaten auch solche, die zusätzlich zur antiinflammatorischen Wirksamkeit noch eine ausgeprägte antiulcerogene sowie tumorhemmende Wirksamkeit besitzen.

Die Herstellung der Arzneimittelspezialitäten erfolgt in üblicher Weise, indem man die Wirkstoffe mit geeigneten Zusätzen, Trägersubstanzen und Geschmackskorrigentien in die gewünschten Applikationsformen wie Tabletten, Dragees, Kapseln, Lösungen, Inhalationsmittel usw. überführt.

Für die orale Anwendung eignen sich insbesondere Tabletten, Dragees und Kapseln, welche beispielsweise 1 bis 250 mg Wirkstoff und 50 mg bis 2 g pharmakologisch unwirksamen Trägers, wie zum Beispiel Laktose, Amylose, Talkum, Gelatine, Magnesiumstearat und ähnliches, sowie die üblichen Zusätze enthalten.

Die nachfolgenden Ausführungsbeispiele dienen zur Erläuterung des erfindungsgemässen Verfahrens.

Beispiel 1

a) 10,1 g 5-Brom-6-nitroindan, 4,1 g Kupfer(I)-chlorid, 7,1 g Kalium-tert.-butanolat und 8,5 g 2,4-Difluorphenol werden in 210 ml tert.-Butanol 7 Stunden gekocht. Kühlen, Verdünnen mit Äther, Filtrieren, Einengen, Aufnehmen des Rückstandes in Äther, Waschen der ätherischen Lösung mit 1 n Salzsäure sowie Trocknen und Einengen ergibt 10,5 g Rohprodukt, das über eine Kieselgelsäule mit Hexan-Ethylacetat chromatographiert wird. Ausbeute 6,3 g an 5-(2,4-Difluorphenoxy)-6-nitroindan vom Schmelzpunkt 65 bis 68°C (aus Hexan).

b) 14,6 g 5-(2,4-Difluorphenoxy)-6-nitroindan werden in 300 ml Dioxan-Ether 1:1 mit 10 g Raney-Nickel und anschliessend bei 40°C mit 4,86 ml Hydrazinhydrat versetzt. Nach weiteren 30 Minuten bei 50°C und 30 Minuten am Rückfluss wird gekühlt, filtriert und eingeengt. Ausbeute 13 g an rohem 6-(2,4-Difluorphenoxy)-5-indanylamin.

c) 13,1 g 6-(2,4-Difluorphenoxy)-5-indanylamin in 60 ml Pyridin werden bei 0°C mit 4,0 ml Methansulfonylchlorid versetzt. Nach 3 Stunden bei 0°C und 16 Stunden bei 20°C wird eingeengt, der Rückstand in Chloroform aufgenommen, die Lösung mit 1 n Salzsäure gewaschen und eingeengt. Umkristallisation des Rückstandes aus Ethanol ergibt 8,1 g N-[6-(2,4-Difluorphenoxy)-5-indanyl]-methansulfonamid vom Schmelzpunkt 85 bis 87°C.

Beispiel 2

3 g N-[6-(2,4-Difluorphenoxy)-5-indanyl]-methansulfonamid in 30 ml Pyridin werden bei 0°C in 10 Minuten unter Stickstoff mit 1,5 ml Essigsäureanhydrid versetzt und 3 Stunden bei 0°C und 13 Stunden bei Raumtemperatur nachgerührt. Es wird eingeengt, der Rückstand in Chloroform aufgenommen, dreimal mit 1 n Salzsäure und einmal mit Wasser ausgeschüttelt, die organische Phase über Calciumsulfat getrocknet, eingeengt und der Rückstand aus Ethanol umkristallisiert.

Ausbeute: 3,1 g N-Acetyl-N-[6-(2,4-difluorphenoxy)-5-indanyl-methansulfonamid vom Schmelzpunkt 160°C.

Beispiel 3

12,8 g 5-Amino-6-(2,4-difluorphenoxy)-1-indanon in 95 ml Pyridin werden bei 0°C mit 8,3 ml Methansulfonylchlorid versetzt. Nach 3 Stunden bei 0°C und 16 Stunden bei 20°C wird eingeengt, der Rückstand in Chloroform aufgenommen, die Lösung mit 1 n Salzsäure gewaschen und eingeengt. Chromatographie des Rückstandes über Kieselgel mit Dichlormethan-Ethylacetat ergibt 1,2 g 6-(2,4-Difluorphenoxy)-5-bis(methylsulfonyl)-amino-1-indanon vom Schmelzpunkt 190°C (aus Toluol), und danach 8,9 g 6-(2,4-Difluorphenoxy)-5-methylsulfonylamino-1-indanon vom Schmelzpunkt 153°C (aus Ethanol).

Die Ausgangssubstanz für diesen Syntheseschritt kann auf zwei Wegen erhalten werden:

Weg 1

a) 13,9 g 6-(2,4-Difluorphenoxy)-5-indanylamin in 93 ml Essigsäure wurden bei 30°C mit 40 ml Acetanhydrid versetzt. Danach wird eine Lösung von 11 g Chromtrioxid in 27 ml Wasser 17 ml Essigsäure bei 50°C zugetropft. Nach weiteren 40 Minuten bei 50°C wird gekühlt, auf Eiswasser gegeben und abgesaugt. Der Rückstand wird über Kieselgel mit Dichlormethan-Ethylacetat chromatographiert. Dabei werden 9 g 5-Acetylamino-6-(2,4-difluorphenoxy)- 1-indanon vom Schmelzpunkt 153°C, danach 4 g des isomeren 6-Acetylamino-5-(2,4-difluorphenoxy)-1-indanon vom Schmelzpunkt 199°C erhalten.

b) 12,9 g 5-Acetylamino-6-(2,4-difluorphenoxy)-1-indanon werden in 210 ml Ethanol mit 22 ml konzentrierter Salzsäure 2 Stunden gekocht. Danach wird eingeengt, der Rückstand mit Wasser und Ammoniaklösung versetzt (pH 8) und der Feststoff 5-Amino-6-(2,4-difluorphenoxy)-1-indanon abgesaugt. Ausbeute 11,1 g vom Schmelzpunkt 132°C.

Weg 2

a) 4,58 g 5-(2,4-Difluorphenoxy)-6-nitroindan und 8,2 g Bis-(dimethylamino)-tert.-butoxy-methan werden in 5 ml Dimethylformamid 60 Minuten bei 140°C gerührt. Einengen im Vakuum ergibt rohes 1-Dimethylaminomethylen-5-(2,4-difluorphenoxy)-6-nitroindan.

b) Dieses wird in Chloroform gelöst, und bei −40°C wird Ozon eingeleitet (12 Minuten, Geschwindigkeit 4,5 g pro Stunde). Nach Einleiten von Stickstoff wird auf Eiswasser gegeben, mit Salzsäure auf pH 3 gebracht, mit Natriumhydrogensulfitlösung gewaschen und eingeengt. Chromatographie des Rückstandes über Kieselgel mit Chloroform ergibt 250 mg 5-(2,4-Difluorphenoxy)-6-nitro-1-indanon vom Schmelzpunkt 145°C (aus Ethanol).

c) Dieses wird in 5 ml Ethanol-Dioxan 1:1 gelöst, 250 mg Raney-Nickel werden hinzugefügt und anschliessend bei 45°C 100 mg Hydrazinhydrat. Nach 30 Minuten am Rückfluss wird gekühlt, filtriert und eingeengt. Ausbeute 240 mg 5-Amino-6-(2,4-difluorphenoxy)-1-indanon vom Schmelzpunkt 153°C (aus Ethanol).

Beispiel 4

2,82 g 6-(2,4-Difluorphenoxy)-5-methylsulfonylamino-1-indanon werden in 30 ml Pyridin mit 1,57 g Acetylchlorid versetzt. Nach 20 Stunden bei 20°C wird eingeengt, mit Wasser versetzt, mit Salzsäure auf pH 6 gebracht und mit Chloroform extrahiert. Der Chloroformextrakt wird neutral gewaschen, eingeengt und der Rückstand mit Toluol-Ethanol 99:1 über Kieselgel chromatographiert.

Ausbeute: 2,50 g 5-(N-Acetyl-N-methylsulfonylamino)-6-(2,4-difluorphenoxy)-1-indanon vom Schmelzpunkt 182°C (aus Ethanol).

Beispiel 5

3,53 g6-(2,4-Difluorphenoxy)-5-methylsulfonyl-amino-1-indanon werden in 35 ml Methanol und 10 ml 1 N Natronlauge gelöst und bei 5°C portionsweise mit 0,8 g Natriumborhydrid versetzt. Nach 16 Stunden bei 20°C wird eingeengt, mit 40 ml Wasser und 26 ml 1 N Salzsäure versetzt und abgesaugt. Umkristallisation aus Ethanol ergibt 3,07 g N-[6-(2,4-Difluorphenoxy)-1-hydroxy-5-indanyl]-methansulfonamid vom Schmelzpunkt 127°C.

Beispiel 6

7,06 g 6-(2,4-Difluorphenoxy)-5-methansulfonylamino-1-indanon werden in 100 ml Methanol und 40 ml Wasser mit 3,40 g Natriumacetat-trihydrat und 4 g Hydroxylaminhydrochlorid 3 Stunden gekocht. Nach Abkühlen wird abgesaugt und getrocknet. Ausbeute 6,16 g N-[1-Hydroxyimino-6-(2,4-difluorphenoxy)-5-indanyl]-methansulfonamid vom Schmelzpunkt 240°C.

Beispiel 7

3,68 g N-[1-Hydroxyimino-6-(2,4-difluorphenoxy)-5-indanyl]-methansulfonamid werden in 100 ml Ethanol gelöst. Die Lösung wird mit Ammoniakgas gesättigt, 1 g Raney-Nickel wird hinzugefügt, und es wird bei 90°C hydriert. Kühlen, Filtrieren, Einengen, Versetzen mit ethanolischer Salzsäure, Einengen und Kristallisation mit Ether ergibt 2,99 g N-[1-Amino-6-(2,4-difluorphenoxy)-5-indanyl]-methansulfonamid, Hydrochlorid, Schmelzpunkt 220°C.

**Patentansprüche**

1. Indanyl-Derivate der allgemeinen Formel I

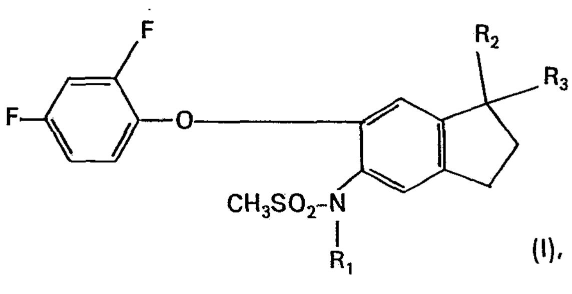

(I),

worin

$R_1$ ein Wasserstoffatom, eine Methansulfonylgruppe, oder eine Acetylgruppe darstellt und worin

$R_2$ und $R_3$ gemeinsam eine Oxogruppe, eine Oximinogruppe oder zwei Wasserstoffatome oder $R_2$ ein Wasserstoffatom und $R_3$ eine Hydroxygruppe oder eine Aminogruppe bedeuten und falls $R_3$ eine Aminogruppe darstellt, deren Salze physiologisch unbedenklicher Säuren.

2. N-[6-(2,4-Difluorphenoxy)-5-indanyl]-methansulfonamid.

3. N-Acetyl-N-[6-(2,4-Difluorphenoxy)-5-indanyl]-methansulfonamid.

4. 6-(2,4-Difluorphenoxy)-5-methylsulfonyl-amino-1-indanon.

5. 5-(N-Acetyl-N-methylsulfonyl-amino)-6-(2,4-difluorphenoxy)-1-indanon.

6. N-[6-(2,4-Difluorphenoxy)-1-hydroxy-5-indanyl]-methansulfonamid.

7. N-[1-Amino-6-(2,4-Difluorphenoxy)-1-indanyl]-methansulfonamid-Hydrochlorid.

8. Pharmazeutische Präparate, gekennzeichnet durch ein oder zwei Indanyl-Derivate gemäss Anspruch 1 bis 7 als Wirkstoff.

9. Verfahren zur Herstellung von Indanyl-Derivaten gemäss Anspruch 1 bis 7, dadurch gekennzeichnet, dass man in an sich bekannter Weise eine Verbindung der allgemeinen Formel II

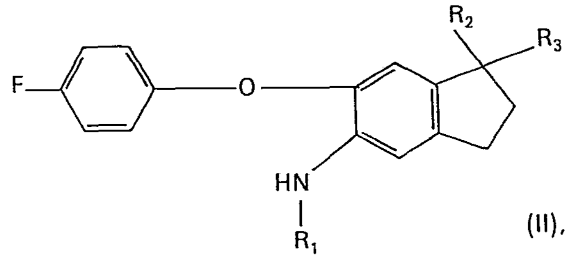

(II),

worin

$R_1$, $R_2$ und $R_3$ die im Anspruch 1 genannte Bedeutung besitzen mit Methansulfonsäurechlorid kondensiert, gegebenenfalls Indanyl-Derivate mit $R_4$ und $R_5$ in der Bedeutung einer Oxogruppe oder einer Oximinogruppe reduziert oder Indanyl-Derivate der allgemeinen Formel I mit $R_1$ in der Bedeutung von Wasserstoff acetyliert.

**Claims**

1. Indanyl derivatives of the general formula I

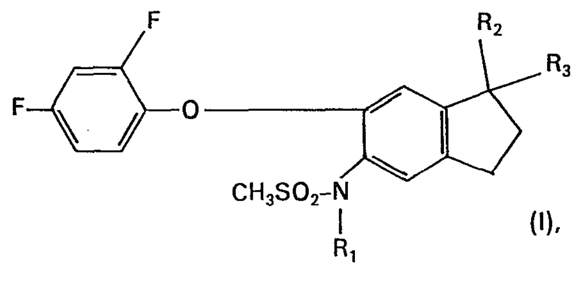

(I),

wherein

$R_1$ represents a hydrogen atom, a methanesulphonyl group or an acetyl group and wherein

$R_2$ and $R_3$ together represent an oxo group, an oximino group or two hydrogen atoms or $R_2$ represents a hydrogen atom and $R_3$ represents a hydroxy group or an amino group and when $R_3$ represents an amino group, their salts with physiologically acceptable acids.

2. N-[6-(2,4-difluorophenoxy)-5-indanyl]-methanesulphonamide.

3. N-acetyl-N-[6-(2,4-difluorophenoxy)-5-indanyl]-methanesulphonamide.

4. 6-(2,4-Difluorophenoxy)-5-methylsulphonyl-amino-1-indanone.

5. 5-(N-acetyl-N-methylsulphonylamino)-6-(2,4-difluorophenoxy)-1-indanone.

6. N-[6-(2,4-difluorophenoxy)-1-hydroxy-5-indanyl]-methanesulphonamide.

7. N-[1-amino-6-(2,4-difluorophenoxy)-1-indanyl]-methanesulphonamide hydrochloride.

8. Pharmaceutical preparations characterised by one or two indanyl derivatives according to claims 1 to 7 as active ingredient.

9. Process for the preparation of indanyl derivatives according to claims 1 to 7 characterised in that one condenses in a known manner a compound of the general formula II

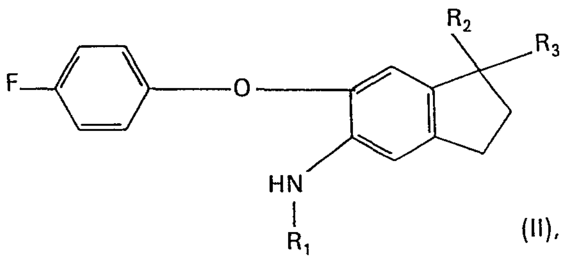

(II),

wherein

$R_1$, $R_2$ and $R_3$ have the meanings set out in claim 1 with methanesulphonyl chloride, reduces in the case of indanyl derivatives where $R_4$ and $R_5$ represent an oxo group or an oximino group, or acetylates indanyl derivatives of the general formula I where $R_1$ represents hydrogen.

**Revendications**

1. Dérivés de l'indane de formule générale I ci-dessous:

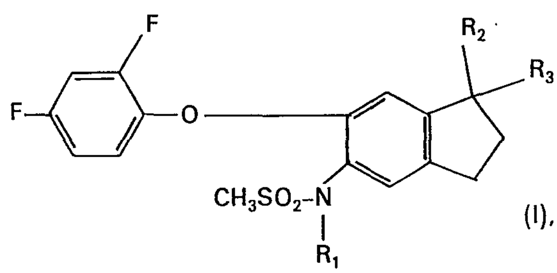

(I),

dans laquelle:

$R_1$ représente un atome d'hydrogène ou un groupe méthanesulfonyle ou acétyle et

$R_2$ et $R_3$ forment ensemble un groupe oxo ou hydroxy-imino ou bien sont deux atomes d'hydrogène, ou encore $R_2$ est un atome d'hydrogène et $R_3$ un hydroxyle ou un groupe amino, et, si $R_3$ est un groupe amino, les sels de ces dérivés formés avec des acides sans inconvénient physiologique.

2. N-[6-(2,4-Difluorophénoxy)-5-indanyl]-méthanesulfonamide.

3. N-Acétyl-N-[6-(2,4-difluorophénoxy)-5-indanyl]-méthane-sulfonamide.

4. 6-(2,4-Difluorophénoxy)-5-méthylsulfonyl-amino-1-indanone.

5. 5-(N-Acétyl-N-méthylsulfonyl-amino)-6-(2,4-difluorophénoxy)-1-indanone.

6. N-[6-(2,4-Difluorophénoxy)-1-hydroxy-5-indanyl]-méthane-sulfonamide.

7. Chlorhydrate de N-[1-amino-6-(2,4-difluorophénoxy)-1-indanyl]-méthane-sulfonamide.

8. Préparations pharmaceutiques caractérisées en ce qu'elles contiennent comme matières actives un ou deux dérivés de l'indane selon l'une quelconque des revendications 1 à 7.

9. Procédé de préparation de dérivés de l'indane selon les revendications 1 à 7, procédé caractérisé en ce que l'on condense, de manière en elle-même connue, un composé de formule générale II:

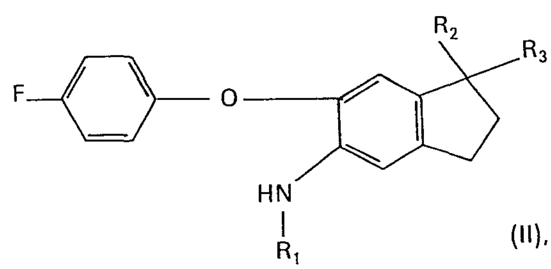

(II),

dans laquelle

$R_1$, $R_2$ et $R_3$ ont les significations données à la revendication 1, avec le chlorure de l'acide méthane-sulfonique, et le cas échéant on réduit les dérivés dans lesquels $R_2$ et $R_3$ forment un groupe oxo ou un groupe hydroxy-imino, ou an acétyle ceux dans lesquels $R_1$ est un atome d'hydrogène.